# EUROPÄISCHE PATENTANMELDUNG

(11) **EP 1 563 823 A2**
(43) Veröffentlichungstag der Anmeldung: **17.08.2005**
(21) Anmeldenummer: 05003098.0
(22) Anmeldetag: 14.02.2005
(51) Int. Cl.: A61K 6/093, A61K 6/10

(54) **Dentalmaterial auf Basis von alkoxysilylfunktionellen Polyethern mit einem Katalysator**

(30) Priorität: 13.02.2004 DE 102004008022
(71) Anmelder: Kettenbach GmbH & CO. KG, 35713 Eschenburg (DE)
(72) Erfinder: Bublewitz, Alexander, Dr., 35745 Herborn (DE); Reber, Jens-Peter, Dr., 58540 Meinerzhagen (DE)
(74) Vertreter: KEIL & SCHAAFHAUSEN

(57) **Zusammenfassung**

Die vorliegende Erfindung betrifft kondensationsvernetzende Dentalmaterialien, insbesondere kondensationsvernetzende Zweikomponenten-Dentalabformmaterialien, auf Basis von alkoxysilylfunktionellen Polyethern enthaltend wenigstens einen alkoxysilylfunktionellen Polyether sowie wenigstens einen Katalysator, wobei der wenigstens eine Katalysator ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2, welche vorzugsweise eine Struktur aufweist, welche nach Deprotonierung der Säure eine Mesomeriestabilisierung der negativen Ladung ermöglicht.

## Beschreibung

Die vorliegende Erfindung betrifft kondensationsvernetzende Dentalmaterialien, insbesondere kondensationsvernetzende Zweikomponenten-Dentalabformmaterialien, auf Basis von alkoxysilylfunktionellen Polyethern, welche insbesondere zur Abdrucknahme geeignet sind, und deren Verwendung. Derartige Materialien werden in der Dentalmedizin bspw. zur Zahnabdrucknahme, Bissregistrierung, Zahnprothesenunterfütterung, als provisorischer und permanenter Dentalzement, provisorisches Verschlussmaterial oder dentales Zahntechnik-Dubliermaterial eingesetzt.

Bekannte kondensationsvernetzende Dentalmaterialien enthalten üblicherweise hydroxylfunktionelle Polymere mit einem Siliconrückgrat, welche in Gegenwart organischer Zinnverbindungen als Katalysatoren, Alkoxysilanen und/oder Kieselsäurestern als Vernetzer und Wasser aushärten. Allerdings sind derartige Materialien aufgrund des Siliconrückgrats der Polymere vergleichsweise hydrophob, so dass diesen zwecks Herabsetzung der Oberflächenspannung und zur Einstellung der erforderlichen Benetzbarkeit erhebliche Anteile an Tensiden zugefügt werden müssen. Ein weiterer Nachteil dieser Zusammensetzungen liegt in dem Einsatz toxikologisch bedenklicher organischer Zinnverbindungen als Katalysator.

Alternativ dazu sind Zweikomponenten-Dentalmaterialien bekannt, welche terminale Alkoxysilylgruppen aufweisende Polymere mit einem hydrophilen Polyetherrückgrat enthalten, die zur Benetzung der feuchten Zahnsubstanz ausreichend hydrophile Eigenschaften aufweisen. Üblicherweise bestehen diese Materialien aus einer Basiskomponente enthaltend alkoxysilylfunktionelle Polyether mit einem mittleren Molekulargewicht von 800 bis 20.000 g/mol, welche synthesebedingt auch Harnstoff- und/oder Urethangruppen aufweisen können, Füllstoffe sowie ggf. weitere Zusatzstoffe, und einer Katalysatorkomponente, welche neben Füll- und ggf. weiteren Hilfsstoffen eine organische und/oder anorganische Säure als Katalysator enthält.

Aus der EP 0 269 819 B1 sind kondensationsvernetzende Zweikomponenten-Dentalmaterialien bekannt, deren Basiskomponente Alkoxysilylendgruppen enthaltende Polyadditionsprodukte mit einer überwiegend linearen Molekülstruktur und einem mittleren Molekulargewicht von 800 bis 20.000 g/mol enthalten, welche einen Gehalt an Polyethergruppen von 25 bis 90 Gew.-%, einen Gehalt an Urethangruppen von 0,5 bis 10 Gew.-%, einen Gehalt an Harnstoffgruppen von 0,5 bis 10 Gew.-% sowie einen Gehalt an terminalen Alkoxysilylgruppen von 1 bis 25 Gew.-%, und, deren Katalysatorkomponente eine Abmischung enthaltend Wasser sowie organische und/oder anorganische Säuren in Gewichtsmengenverhältnissen (Wasser/Säure) von 1:0,01 bis 1:40, aufweisen. Allerdings ist die Synthese der in der Basiskomponente enthaltenden funktionellen Polyetherpolymere sehr aufwendig und teuer. Ein weiterer Nachteil dieser Dentalmaterialien liegt in dem Einsatz säurehaltiger Katalysatoren. Zum einen können durch die Säurekatalysatoren bei der Abformung im Patientenmund die Mundschleimhaut und der Zahnschmelz durch Säureätzung geschädigt werden. Zudem erlauben diese Systeme keinen Zusatz an stickstoffbasenhaltigen Substanzen, wie Adstringentien, bspw. Epinephrine, oder anderen säurelabilen therapeutischen Zusätze, da diese durch den Säurekatalysator aufgrund von Protonierung oder Spaltung inaktiviert werden. Ferner erfordert der Einsatz von Säuren bei der Produktion der Dentalmaterialien entsprechende Sicherheitsvorkehrungen.

In der EP 1 226 808 A2 werden kondensationsvernetzende Zweikomponenten-Dentalmaterialien bestehend aus einer Basis- und Katalysatorkomponente offenbart, deren Basiskomponente alkoxysilylfunktionelle Polyether mit linearer oder verzweigter Hauptkette und einem mittleren Molekulargewicht von 800 bis 20.000 g/mol enthalten, welche einen Gehalt an Polyethergruppen von 20 bis 95 Gew.-%, einen Gehalt an terminalen Alkoxysilylgruppen von 0,2 bis 25 Gew.-% sowie ggf. einen Gehalt an Urethangruppen oder Harnstoffgruppen von bis zu 10 Gew.-%, und, deren Katalysatorkomponente eine Mischung enthaltend Wasser sowie organische und/oder anorganische Säuren in Gewichtsmengenverhältnissen von 1:0,01 bis 1:40, aufweisen. Vorzugsweise enthält die Katalysatorkomponente p-Toluolsulfonsäurehydrat als Katalysator sowie ein Polyetherdiol und weitere Zusatzstoffe, wie Füllstoffe, Paraffin, Emulgator und dergl. Zwar sind die in diesen Dentalmaterialien eingesetzten funktionellen Polyetherpolymere gegenüber den zuvor genannten einfacher und kostengünstiger zu synthetisieren und zeichnen sich durch eine bessere Abbindekinetik aus. Allerdings machen auch diese Dentalmaterialien von säurehaltigen Katalysatoren Gebrauch, so dass einerseits die Gefahr der Schädigung der Mundschleimhaut sowie des Zahnschmelzes bei der Abformung im Patientenmund besteht und diesen Materialien zudem keine säurelabilen therapeutischen Zusätze zugesetzt werden können. Ein weiterer Nachteil der Systeme liegt in deren geringeren Lagerstabilität. Eine unabhängig von der Lagerungszeit gleichbleibende Abbindezeit ist allerdings eine der wichtigsten Anforderungen an ein dentales Abformmaterial.

In der WO 99/48942 wird vorgeschlagen, zur Vernetzung von als Kleb- oder Dichtstoffen einzusetzenden und Polyethergruppen aufweisenden Polyurethanen metalllorganische Verbindungen, wie Eisen- oder Zinnverbindungen, bspw. Zinn(II)oktat, oder tertiäre Amine, wie Triethylamin, einzusetzen. Ein Nachteil dieser Katalysatoren liegt jedoch in deren hohen Toxizität, so dass bei der Produktion der Materialien geeignete sicherheitstechnische Vorkehrungen zu treffen sind und eine Verwendung der Materialien als Dentalabformmassen nicht ohne weiteres möglich ist. Zudem sind insbesondere tertiäre Amine geruchsintensiv, weshalb deren Einsatz in Dentalmaterialien unerwünscht ist. Zudem sind die in diesen Materialien eingesetzten Polyurethane aufgrund deren hohen Anteil an Urethangruppen pro Molekül durch starke intermolekulare Wechselwirkungen gekennzeichnet, was bei gegebener Kettenlänge der Moleküle zu einer verglichen mit alkoxysilylfunktionellen Polyethern, die pro Molekül nur zwei Urethangruppen aufweisen, zu einer erhöhten Viskosität der Materialien führt, weswegen in diesen Materialien weniger Füllstoffe eingesetzt werden können, was wiederum erhöhte Herstellungskosten bedingt.

Ferner ist aus der EP 1 402 873 A1 eine zweikomponentige, bei Raumtemperatur zu einem elastomeren Material abbindende Zubereitung bekannt, bei welcher in der Katalysator-Komponente B eine organische oder anorganische Säure und in der Basiskomponente A ein silanfunktionalisiertes Polyetherderivat sowie eine antacid wirkende Verbindung enthalten ist, wobei die antacid wirkende Verbindung aus der aus basischen oder amphoteren Oxiden, Hydroxiden, Carbonaten, Carboxylaten und basischen organischen Verbindungen mit N, As, O, P, S oder Sb als Heteroatom sowie stickstoffhaltigen organischen Verbindungen mit Isocyanat-, Epoxid-, Carbodiimid- oder Aziridinogruppen bestehenden Gruppe ausgewählt ist.

Aufgabe der vorliegenden Erfindung ist es daher, ein hydrophiles kondensationsvernetzendes Dentalmaterial, insbesondere ein kondensationsvernetzendes Zweikomponenten-Dentalabformmaterial, auf Basis von Alkoxysilylpolyethern zur Verfügung zu stellen, welches lagerstabil ist, insbesondere eine konstante Reaktionskinetik auch nach mindestens 18 Monaten Lagerungszeit gewährleistet, eine gute Biokompatibilität aufweist, insbesondere geruchs- sowie geschmackneutral ist, und toxikologisch möglichst unbedenkliche Inhaltsstoffe aufweist.

Erfindungsgemäß wird diese Aufgabe durch ein kondensationsvernetzendes Dentalmaterial der Zusammensetzung gemäß Patentanspruch 1 gelöst.

Überraschenderweise konnte im Rahmen der vorliegenden Erfindung gefunden werden, dass die erfindungsgemäß einzusetzenden Salzkatalysatoren eine gute katalytische Aktivität für Kondensationsreaktionen aufweisen und diese daher hervorragend dafür geeignet sind, in kondensationsvernetzenden Dentalmaterialien auf Basis von alkoxysilylfunktionellen Polyethern als Katalysator eingesetzt zu werden. Die erfindungsgemäßen Dentalmaterialien weisen nicht nur eine für Dentalmaterialien geeignete Reaktionskinetik auf, sondern insbesondere auch eine praxisgerechte Verarbeitungs- und Abbindezeit. Dabei zeichnen sich diese Katalysatoren gegenüber den zu diesem Zweck bisher bekannten Substanzen, wie metallorganischen Verbindungen und tertiären Aminen, durch eine gute Biokompatibilität aus und erfordern bei der Herstellung der Dentalmaterialien weniger rigide Sicherheitsvorkehrungen. Insbesondere kann auf den Einsatz schwermetallhaltiger Katalysatoren, wie zinn-, zink- oder bleiorganische Katalysatorverbindungen, verzichtet werden. Ein weiterer Vorteil der erfindungsgemäß eingesetzten Salzkatalysatoren insbesondere gegenüber den aus dem Stand der Technik bekannten primären, sekundären und tertiären Aminen liegt in deren Geruchs- und Geschmacksneutralität, was für ein dentales Abformmaterial eine wichtige Eigenschaft ist, um die Patientenakzeptanz zu erreichen und bspw. Würgereaktionen des Patienten bei der Applikation zu vermeiden. Zudem war es für den Fachmann unerwartet, dass derartige Dentalmaterialien auch nach mehrmonatiger Lagerung eine gleich bleibende Reaktionskinetik, insbesondere eine konstante Verarbeitungs- und Abbindezeit, aufweisen. Dies liegt u.a. darin begründet, dass die erfindungsgemäß eingesetzten Katalysatorsalze während der Lagerzeit und nach dem Aushärten keine Neben- oder Abbaureaktionen mit anderen Inhaltsstoffen, wie bspw. mit den Füllstoffen, mit dem ggf. als Pastenbildner eingesetzten Polyether oder dem Alkoxysilylpolyether, eingehen. Abgesehen davon ist der Einsatz eines Salzes als Katalysator verglichen mit dem einer freien Säure und einer freien Base, wie einem tertiären Amin, auch deshalb vorteilhaft, weil das Salz einen vergleichsweise moderaten pH-Wert aufweist, wodurch eine gute Verträglichkeit der erfindungsgemäßen Dentalmaterialien mit der Mundschleimhaut und dem Zahnschmelz gewährleistet wird, so dass bei der Applikation keine Verätzungen oder Irritationen auftreten.

Die erfindungsgemäßen Dentalmaterialien können sowohl als Einkomponentenals auch als Zweikomponentenmaterial formuliert sein. Während die Formulierung der Einkomponenten-Dentalmaterialien möglichst absolut wasserfrei sein muss, um eine Reaktion der alkoxysilylfunktionellen Polyether während der Lagerung zu verhindern, und die Reaktion der alkoxysilylfunktionellen Polyether nach der Applikation der Materialien auf dem abzuformenden Gegenstand durch Luftfeuchtigkeit initiiert wird, ist der Katalysatorkomponente des erfindungsgemäßen Zweikomponenten-Dentalmaterials vorzugsweise Wasser zugesetzt. Vorzugsweise werden die erfindungsgemäßen Zweikomponenten-Dentalmaterialien so formuliert, dass die

### Basiskomponente A

a) wenigstens einen alkoxysilylfunktionellen Polyether,
und die Katalysatorkomponente B
b) wenigstens einen Katalysator und
c) Wasser
enthält, wobei der wenigstens eine Katalysator b) ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2.

Gemäß einer ersten besonderen Ausführungsform der vorliegenden Erfindung ist der wenigstens eine Katalysator ein Salz, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2, wobei die Säure eine Struktur aufweist, welche nach Deprotonierung der Säure eine Mesomeriestabilisierung der negativen Ladung ermöglicht. Mesomeriestabilisierung im Sinne der vorliegenden Erfindung bedeutet im Einklang mit dem allgemeinen Lehrbuchwissen, dass sich für die deprotonierte Säure wenigstens zwei Grenzstrukturen formulieren lassen, in denen die negative Ladung an unterschiedlichen Atomen lokalisiert ist, bzw., dass in der deprotonierten Säure π-Elektronenen delokalisiert sind, was zu einer Stabilisierung der deprotonierten Form führt.

Unabhängig davon, ob das erfindungsgemäße Dentalmaterial als Ein- oder Zweikomponentensystem formuliert ist, ist das eingesetzte Katalysatorsalz vorzugsweise gebildet aus wenigstens einer Säure mit einem in Wasser gemessenen pKs-Wert von kleiner 1 und besonders bevorzugt von kleiner gleich 0,7 und/oder wenigstens einer Base mit einem in Wasser gemessenen pK_{BH+}-Wert zwischen 1 und 7, besonders bevorzugt zwischen 2 und 6 und ganz besonders bevorzugt zwischen 3 und 6.

Gemäß einer weiteren besonderen Ausführungsform der vorliegenden Erfindung ist das wenigstens eine Katalysatorsalz gebildet aus einer aus der aus p-Toluolsulfonsäure, Fluorsulfonsäure, Trifluormethansulfonsäure, Fluoroschwefelsäure, 4-Sulfophthalsäure, Trichloressigsäure, Trifluoressigsäure, Benzolsulfonsäure und Kombinationen hiervon bestehenden Gruppe ausgewählten Säure und/oder einer aus der aus Pyrrolderivaten, Dimethylanilin, Pyridin, 2,4,6-N,N-Pentamethylanilin, N,N-Dimethylanilin, Phenetedin, Acridin, Phenanthridin, Chinolin, Isochinolin, 2-Amino-4,6-dimethylpyrazin, 4,6-Dimethylpyridinamin, 3-Methylpyridin(3-picolin), 4-Phenylpyridin, 4-Vinylpyridin, Pyridazin, 2-Ethylpyridin, 2-Butylpyridin, 1,7-Phenanthrolin, 2-Aminopyrimidin, 2-Isopropylpyridin, 2-Vinylpyridin, 2-N,N-Dimethyl-aminopyridin, Chinazolin, 4-Chloropyridin, Phenazin, 4-Acetylpyridin, Methylnicotinat, 3-Benzoylpyridin, 2,2'-Bipyridin, 2-Phenylpyridin, 2-tert-Butylpyridin, Pyrimidin, 3-lodopyridin, 3-Fluoropyridin, 3-Chloropyridin, 3-Bromopyridin, Pyrazin, 7,8-Benzochinolin, 2-Chloropyridin, 4-Cyanopyridin und Kombinationen hiervon bestehenden Gruppe ausgewählten Base. Besonders bevorzugt wird als Katalysator ein Salz von p-Toluolsulfonsäure mit Pyridin oder ein Salz von p-Toluolsulfonsäure mit 2,4,6-N,N-Pentamethylanilin eingesetzt.

In Weiterbildung des Erfindungsgedankens wird vorgeschlagen, dass die in den erfindungsgemäß einzusetzenden Katalysatorsalzen vorgesehenen Kationen und/oder Säureanionen Alkoxysilylgruppen aufweisen. Dadurch wird erreicht, dass das Katalysatorsalz nach Aushärten des Dentalmaterials in der Polyethermatrix eingebunden ist und aus dem dentalen Abformmaterial nicht mehr herausgelöst werden kann.

Erfindungsgemäß können die Dentalmaterialien als Katalysator eines oder, in beliebiger Kombination miteinander, mehrere der zuvor genannten Salze enthalten. Vorzugsweise enthält das Dentalmaterial nur eines der zuvor genannten Salze als Katalysator, wobei besonders bevorzugt neben dem einen oder mehreren erfindungsgemäß einzusetzenden Salzen keine weiteren Katalysatoren, insbesondere keine metallorganischen Metallsalze, tertiären Amine oder freie Säuren, eingesetzt werden.

Vorzugsweise wird in dem erfindungsgemäßen Dentalmaterial ein Katalysatorsalz b) mit einem in Wasser (Ampuwa, pH 5,8) gemessenen pH-Wert zwischen 1 und 7, besonders bevorzugt zwischen 2 und 6 und ganz besonders bevorzugt zwischen 2 und 5 eingesetzt.

Wie der Fachmann erkennt hängt die Menge an einzusetzendem Katalysatorsalz u.a. von der Löslichkeit des Salzes in der eingesetzten Polyethermatrix ab. Vorzugsweise beträgt die Menge an einzusetzendem Katalysatorsalz, bezogen auf die Gesamtmischung des Dentalmaterials, 0,0005 bis 0,5 mmol/g, besonders bevorzugt 0,0005 bis 0,25 mmol/g und ganz besonders bevorzugt 0,0005 bis 0,05 mmol/g. Selbstverständlich muss das eingesetzte Katalysatorsalz eine Mindestlöslichkeit in der eingesetzten Polyethermatrix aufweisen, um überhaupt katalytisch wirken zu können.

Um die Menge an einzusetzendem Katalysatorsalz möglichst gering zu halten, wird in Weiterbildung des Erfindungsgedankens vorgeschlagen, in dem Dentalmaterial ein Katalysatorsalz mit einer hinreichend hohen Löslichkeit in dem Polyethermaterial, d.h. mit einer ausreichenden katalytischen Aktivität, einzusetzen, wobei die katalytische Aktivität im Sinne der vorliegenden Patentanmeldung durch die Aushärtezeit nach ISO 4823 (Ausgabe 1992), bestimmt durch Rückstellung nach Verformung, charakterisiert ist. Vorzugsweise wird ein Katalysatorsalz eingesetzt, dass in einer Polyethermatrix umfassend als Struktureinheit Polytetrahydrofuran, Polyethylenglycol und besonders bevorzugt Polypropylenglycol sowie deren Mischungen und Copolymerisate eine Aushärtezeit von kleiner gleich 30 min, besonders bevorzugt kleiner gleich 15 min für eine Zahntechnik-Dubliermasse bzw. eine Aushärtezeit von kleiner gleich 15 min, besonders bevorzugt kleiner gleich 10 min, ganz besonders bevorzugt kleiner gleich 7 min und höchst bevorzugt kleiner gleich 6 min für eine dentale Abformmasse ergibt.

Wenn das erfindungsgemäße Dentalmaterial als Einkomponentenmaterial formuliert ist, sollte es möglichst absolut wasserfrei sein, um eine Reaktion der alkoxysilylfunktionellen Polyether während der Lagerung zu vermeiden.

In dem Fall, dass das Dentalmaterial als Zweikomponentenmaterial formuliert ist, enthält die Katalysatorkomponente B vorzugsweise Wasser c), wohingegen die Basiskomponente A möglichst absolut wasserfrei ist. Vorzugsweise enthält die Katalysatorkomponente B des erfindungsgemäßen Zweikomponenten-Dentalmaterials, bezogen auf die Gesamtmischung, 0,005 bis 3 mmol/g, besonders bevorzugt 0,01 bis 2 mmol/g und ganz besonders bevorzugt 0,02 bis 1 mmol/g an Wasser.

Vorzugsweise enthält das erfindungsgemäße Dentalmaterial wenigstens einen verstärkenden Füllstoff d₁) und/oder wenigstens einen nicht-verstärkenden Füllstoff d₂). Bei Formulierung als Zweikomponenten-Material kann die Basiskomponente A wenigstens einen der vorgenannten Füllstoffe enthalten, wobei vorzugsweise sowohl in der Basiskomponente A und auch in der Katalysatorkomponente B wenigstens ein verstärkender Füllstoff und/oder wenigstens ein nicht-verstärkender Füllstoff vorgesehen ist.

Als verstärkende Füllstoffe d₁) eignen sich insbesondere hochdisperse, aktive Füllstoffe mit einer BET-Oberfläche von wenigstens 50 m²/g und/oder einer Primärpartikelgröße von kleiner gleich 100 nm, besonders bevorzugt kleiner gleich 80 nm. Besonders geeignet sind solche mit einer Primärpartikelgröße im Nanometerbereich, welche als Aggregate und/oder Agglomerate vorliegen können. Bevorzugt ist der wenigstens eine verstärkende Füllstoffe d₁) eine Substanz ausgewählt aus der Gruppe, welche aus Aluminiumhydroxid, Zinkoxid, Titandioxid, Zirkoniumoxid, Siliciumdioxid sowie gefällter und/oder pyrogener Kieselsäure besteht. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff d₁) in Form von Nanopartikeln, als faser- oder blättchenförmiger Füllstoff, bspw. mineralischer, faserförmiger Füllstoff, oder als synthetischer, faserförmiger Füllstoff vor.

In Weiterbildung des Erfindungsgedankens wird vorgeschlagen, bei Formulierung als Zweikomponentenmaterial vorzugsweise in der Basiskomponente A, verstärkende Füllstoffe d₁) vorzusehen, die einen Wassergehalt von maximal 0,5 Gew.-%, besonders bevorzugt von maximal 0,3 Gew.-%, ganz besonders bevorzugt von maximal 0,15 Gew.-% aufweisen und höchst bevorzugt absolut bzw. im Wesentlichen wasserfrei sind, wobei der Wassergehalt über Karl-Fischer Titration bestimmt wird.

Gemäß einer weiteren besonderen Ausführungsform der vorliegenden Erfindung, weist der wenigstens eine verstärkende Füllstoff d₁) mit einer BET-Oberfläche von größer 50 m²/g in der Basiskomponente A einen pH-Wert von 4 bis 11, bevorzugt von 5 bis 9 und besonders bevorzugt von 5,0 bis 8,5, auf.

Bei Formulierung als Zweikomponentensystem enthält vorzugsweise die Basiskomponente A, bezogen auf die Komponente A, 0 bis 50 Gew.-%, besonders bevorzugt 0,1 bis 40 Gew.-% und ganz besonders bevorzugt 0,1 bis 30 Gew.-%, und die Katalysatorkomponente B, bezogen auf die Komponente B, 0 bis 50 Gew.-%, besonders bevorzugt 0,1 bis 40 Gew.-% und ganz besonders bevorzugt 0,1 bis 30 Gew.-% wenigstens eines verstärkenden Füllstoffs d₁).

Als nichtverstärkende Füllstoffe d₂) eignen sich prinzipiell dieselben Substanzen wie für die verstärkende Füllstoffe b₁), wobei die nichtverstärkenden Füllstoffe jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugt ist der wenigstens eine nichtverstärkende Füllstoff d₂) eine Substanz ausgewählt aus der Gruppe, welche aus Erdalkalimetalloxiden, Erdalkalimetallhydroxiden, Erdalkalimetallfluorid, Erdalkalimetallcarbonaten, Calciumapatit (Ca₅[(F, Cl, OH, ½CO₃) | (PO₄)₃], insbesondere Calciumhydroxylapatit (Ca₅[(OH) | (PO₄)₃]), Titandioxid, Zirkoniumoxid, Aluminiumhydroxid, Siliciumdioxid, gefällter Kieselsäure und Calciumcarbonat besteht. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe d₂) eine mittlere Korngröße von größer als 0,1 µm (Ullmann Encyclopädie der Technischen Chemie, Band 21, Seite 523) auf.

In Weiterbildung des Erfindungsgedankens wird bei Formulierung des Dentalmaterials als Zweikomponentensystem vorgeschlagen, dass der wenigstens eine nichtverstärkende Füllstoff d₂) in der Basiskomponente A einen Wassergehalt von maximal 0,5 Gew.-%, besonders bevorzugt maximal 0,1 Gew.-%, ganz besonders bevorzugt maximal 0,05 Gew.-% aufweist und höchst bevorzugt absolut bzw. im Wesentlichen wasserfrei ist.

Gemäß einer besonderen Ausführungsform der vorliegenden Erfindung weist der wenigstens eine nichtverstärkende Füllstoff d₂) in der Basiskomponente A einen pH-Wert von 4 bis 11, bevorzugt von 5 bis 9 und besonders bevorzugt von 5,0 bis 8,5 auf.

Vorzugsweise enthält die Basiskomponente A des erfindungsgemäßen Dentalmaterials, bezogen auf die Komponente A, 0 bis 80 Gew.-%, besonders bevorzugt 0,05 bis 75 Gew.-% und ganz besonders bevorzugt 0,1 bis 70 Gew.-%, und die Katalysatorkomponente B, bezogen auf die Komponente B, 0 bis 80 Gew.-%, besonders bevorzugt 0,05 bis 75 Gew.-% und ganz besonders bevorzugt 0,1 bis 70 Gew.-% wenigstens eines nichtverstärkenden Füllstoffs d₂).

In Weiterbildung des Erfindungsgedankens wird vorgeschlagen, dass bei Formulierung als Zweikomponentenmaterial die in der Katalysatorkomponente B enthaltenen verstärkenden und/oder nichtverstärkenden Füllstoffe einen pH-Wert zwischen 2,0 und 7,0 und ganz besonders bevorzugt solche mit einem pH-Wert zwischen 3,0 und 7,0 aufweisen.

Insgesamt beträgt der Gesamtgehalt an Füllstoffen sowohl bei der Formulierung des Dentalmaterials als Ein- als auch als Zweikomponentensystem, bezogen auf die Gesamtmischung, 0 bis 80 Gew.-%, bevorzugt 0,01 bis 80 Gew.-%, besonders bevorzugt 0,1 bis 75 Gew.-% und ganz besonders bevorzugt 0,2 bis 70 Gew.-%.

Als alkoxysilylfunktionelle Polyether a) können prinzipiell alle Polyether enthaltend Alkoxysilylgruppen eingesetzt werden, wobei das Polyetherrückgrat linear und/oder verzweigt und beispielsweise aus Polyethylenoxid, Polypropylenoxid, Polytetrahydrofuran und/oder deren Copolymeren aufgebaut sein kann, wobei diese Monomere statistisch, blockweise oder in einer taktischen Ordnung vorliegen können. Als Starter für die Polyether und/oder Copolymere können ein- oder mehrwertige Alkohole verwendet werden, wie bspw. Methanol, Butanol, Glycerin, Trimethylpropan, Pentaerythrit und Sorbitol. Beispielsweise können Copolymere aus Polytetrahydrofuran mit Polyethylenoxid oder aus Polyethylenoxid und Polypropylenoxid eingesetzt werden, wobei reines Polypropylenoxid besonders bevorzugt ist. Ferner bevorzugt sind Polyether mit seitenständigen Alkylgruppen, wobei jede oder wenigstens jede zehnte Monomerstruktureinheit eine seitenständige Alkylgruppe trägt. Geeignete Handelsprodukte sind Acclaim 4200, Acclaim 6320N, Acclaim 12200, Acclaim 8200 und Acclaim 6300 der Bayer AG, Polyglycol P41/300 und Polyglycol P41/3000 (Clariant) sowie Poly-(ethylenglycol-ran-propylenglycol) (Aldrich). Bevorzugt weisen die Polyether a) ein zahlengemitteltes Molekulargewicht von 500 bis 25.000 g/mol und besonders bevorzugt von 5.000 bis 20.000 g/mol auf.

Ein Auswahlkriterium für den erfindungsgemäß geeigneten Polyether ist neben der Kettenlänge (Elastizität), der Alkoxysilylfunktionalisierung (Aushärte-Kinetik) und der Anzahl der Urethan-/Harnstoffgruppen (Viskosität, Rheologie) die Hydrophilie des Polyethers, die über die Anzahl, Struktur und Polarität der Monomerwiederholungseinheiten des Polyetherpolymers bestimmt wird. Die Hydrophilie für ein Dentalabformmaterial muss einerseits ausreichend hoch sein, um ein gutes Anfließen an feuchte Zahnsubstanz sicherzustellen (niedrige Kontaktwinkel), aber andererseits darf das Material nicht zu hydrophil sein, da sonst Wasser, Feuchtigkeit oder Speichel während der Abdrucknahme bzw. beim Desinfizieren bzw. beim Ausgießen mit Gips zur Quellung führt und damit die erforderliche Dimensionsgenauigkeit nicht mehr gegeben ist. Die Hydrophilie des Polyethers ist darüber hinaus auch unter anderem für die Löslichkeit des erfindungsgemäßen Katalysators mit verantwortlich.

Gemäß einer besonderen Ausführungsform der vorliegenden Erfindung weist der wenigstens eine alkoxysilylfunktionelle Polyether einen Gehalt an Polyethergruppen zwischen 5 und 30 mmol/g und besonders bevorzugt zwischen 10 und 25 mmol/g auf.

Vorzugsweise ist die Alkoxysilylstruktureinheit bzw. sind die Alkoxysilylstruktureinheiten des Polyethers a), bezogen auf das Polymerrückgrat, terminal angeordnet und fallen unter die allgemeine Formel I

- SiR⁵R⁶R⁷

worin R⁵, R⁶ und R⁷ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

In Weiterbildung des Erfindungsgedankens wird vorgeschlagen, dass der wenigstens eine Polyether a) einen Alkoxygruppengehalt von 0,02 bis 12 mmol/g, besonders bevorzugt von 0,04 bis 6 und ganz besonders bevorzugt von 0,04 bis 3 mmol/g, aufweist.

Durch die Art und Anzahl der Alkoxygruppen pro Siliziumatom lässt sich die Kondensationskinetik und damit der Verarbeitungs- und Abbindezeit des Dentalmaterials einstellen. Bevorzugt werden diese Parameter derart gewählt, dass die Verarbeitungszeit 30 Sekunden bis 3 Minuten, besonders bevorzugt zwischen 1 und 2,5 Minuten und ganz besonders bevorzugt zwischen 1,5 und 2 Minuten und/oder die nach ISO 4823 (Ausgabe 1992) bestimmte Abbindezeit im Patientenmund (sog. Mundverweildauer) maximal 15 Minuten, besonders bevorzugt maximal 10 Minuten, ganz besonders bevorzugt maximal 7 Minuten und höchst bevorzugt 6 Minuten beträgt.

Vorzugsweise weist der wenigstens eine Polyether a) als dritte Struktureinheit (neben den terminalen Alkoxygruppen und den Polyethergruppen) jeweils an den terminalen Alkoxysilylgruppen angeordnete Alkylenspacer, welche bevorzugt C₁-C₆-Alkylgruppen, besonders bevorzugt C₁-C₃-Alkylgruppen, ganz besonders bevorzugt Ethylengruppen und/oder Methylengruppen und höchst bevorzugt Methylengruppen sind, auf.

Zudem kann der wenigstens eine Polyether a) als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweisen. Insbesondere dann, wenn der wenigstens eine Polyether a) als vierte Struktureinheit Harnstoff- und/oder Urethangruppen aufweist, ist eine Methylengruppe als Spacer bevorzugt. Durch den Einsatz solcher α-aktivierter Alkoxysilylpolyether werden hydrophile, lagerstabile Zweikomponenten-Dentalabformmassen erhalten, welche mit einem erfindungsgemäß als Katalysator einzusetzenden Salz überraschend schnell durch Kondensationsreaktion vernetzen. Insgesamt sollte der Gehalt an Urethan- bzw. Harnstoffgruppen pro Molekül so gering wie möglich gehalten werden, um die intermolekularen Wechselwirkungen zwischen den einzelnen Polyetherketten zu minimieren, um die durch den Polyetherzusatz begründete Viskosität so gering wie möglich zu halten, was den Zusatz höherer Mengen an Füllstoffen in dem Dentalmaterial ermöglicht und somit mehr Formulierungsfreiräume und kostengünstigere Rezepturen einräumt.

Im Rahmen der vorliegenden Erfindung hat es sich als vorteilhaft erwiesen, Polyether, die innerhalb der Polyetherkette keine Urethan- oder Harnstoffgruppen enthalten und die an jedem Kettenende höchstens/nicht mehr als eine Urethan- oder Harnstoffgruppe und höchstens/nicht mehr als eine (Alkoxy)silylgruppe und höchstens/nicht mehr als eine Methylenspacergruppe tragen, einzusetzen. Der Einsatz dieser Polyether führt verglichen mit den im Stand der Technik eingesetzten Polyurethan-Alkoxysilylpolyethern zu Formulierungen mit niedrigerer Viskosität, so dass den Dentalmaterialien mehr Füllstoffe zugegeben werden können, was zu einer Reduzierung der Herstellkosten führt.

Gemäß einer weiteren besonderen Ausführungsform der vorliegenden Erfindung sind die einzelnen Struktureinheiten des wenigstens einen Polyethers a) folgendermaßen angeordnet: 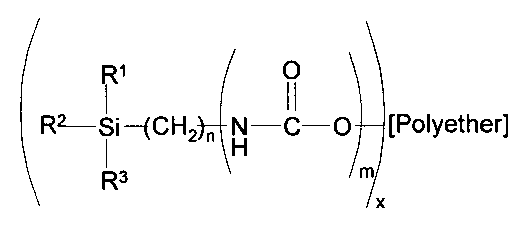 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist,
oder 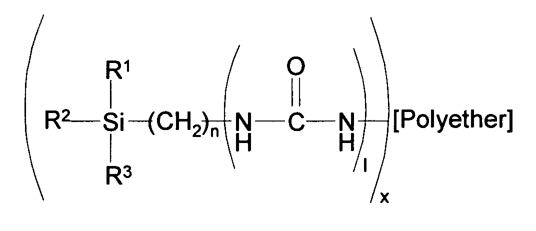 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie I=0 oder 1, besonders bevorzugt I=1 ist.

Gemäß einer weiteren besonderen Ausführungsform der vorliegenden Erfindung ist der Alkylspacer in den vorgenannten Struktureinheiten Methylen (n=1).

Die Herstellung dieser alkoxysilylfunktionellen Polyether ist bekannt und wird beispielsweise in der DE 101 04 079 A1, EP 0 629 819 B1, DE 101 39 132, US 4,906,707, EP 0 372 561 A1, EP 1 303 560 A1 und EP 0 170 865 B1, welche hiermit als Referenz eingeführt und als Teil der Offenbarung gelten, beschrieben. Beispiele für kommerziell erhältliche und im Rahmen der vorliegenden Erfindung geeignete Polyether sind MS Polymer S 203H, MS Polymer S 303H (Kaneka), Polymer XP ST55, ST50, ST51, ST53 (Hanse), SLM 414000 (Wacker), SLM 414001 (Wacker), Baycoll XP 2458 und Desmoseal XP 2447 (Bayer AG), wobei der unter dem Handelsnamen SLM 414000 vertriebene Dimethoxy(methyl)silylmethylcarbamat-terminierte Polyether : 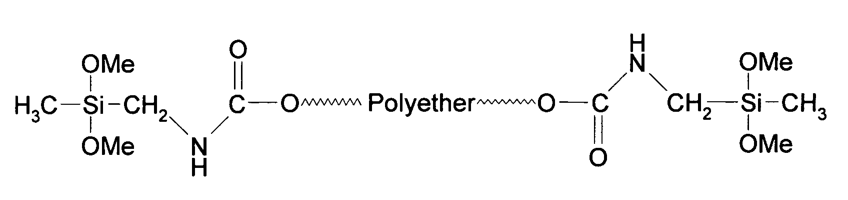 und Dimethoxy(methyl)silylmethylharnstoff-terminierte Polyether: 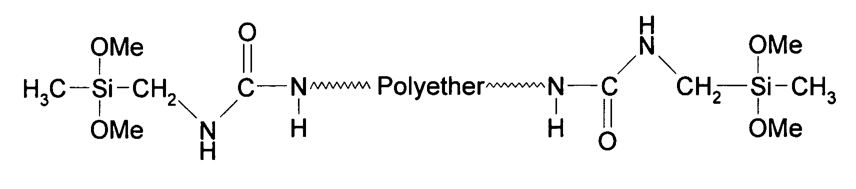 besonders bevorzugt sind.

In Weiterbildung des Erfindungsgedankens wird vorgeschlagen, dem erfindungsgemäßen Dentalmaterial ein oder mehrere der folgenden Zusatz- bzw. Hilfsstoffe zuzusetzen:
f) Thixotropierungsmittel,
g) Wasserfänger,
h) Pastenbildner,
i) Tensid,
j) Wirkstoff,
k) Weichmacher,
l) optische Abtastung ermöglichende Substanz,
m) Geschmacks- und/oder Geruchsstoff,
n) Diagnostik ermöglichende Substanz,
o) Fluoridisierungsmittel,
p) Bleichsubstanz,
q) Desensibilisierungsmittel,
r) Haftverbundvermittler,
s) Farbstoff,
t) Indikator,
u) Stabilisator (Antioxidanz, Radikalfänger).

Dem erfindungsgemäßen Dentalmaterial können optional Thixotropierungsmittel f) zugesetzt werden, wobei sich zu diesem Zweck insbesondere hochmolekulare Polyether, wie Polyethylenglykol, Polyethylenglykol- Polypropylenglykol-Copolymere, Poly-Tetrahydrofuran, Kohlenwasserstoffwachse, Amidwachse, Triglyceride, Kieselsäuren und Silicate als besonders geeignet erwiesen haben.

Gemäß einer besonderen Ausführungsform der vorliegenden Erfindung weisen die Dentalmaterialien, wenn als Zweikomponentensystem formuliert vorzugsweise in der Basiskomponente A, wenigstens einen Wasserfänger g) auf, welcher besonders bevorzugt aus der Gruppe, welche aus Alkoxysilanen, Titanaten, wie Tetraisopropyltitanat, Zirkonaten, wie Tetrapropylzirkonat, Zeolithen, Aluminiumsulfat, wasserfreiem Calciumsulfat (bspw. Drierite®), Blaugel und/oder Oxazolidinen besteht, ausgewählt ist.

In Weiterbildung des Erfindungsgedankens wird vorgeschlagen, als Wasserfänger g) ein oder mehrere funktionelle Alkoxysilane einzusetzen, da durch solche Verbindungen zusätzlich die Vernetzungsgeschwindigkeit, die Struktur und die Eigenschaften des resultierenden Elastomers eingestellt werden können. Bevorzugt ist das wenigstens eine funktionelle Alkoxysilan eine Verbindung der allgemeinen Formel II

R⁸₄₋ₓ-Si-R⁹ₓ

mit R⁸=H, Alkyl, Alkenyl, -(CH₂)ₙ-Z, wobei n=1 bis 6,
R⁹=Alkoxy,
Z=NH₂, NHR, NR₂, wobei R=Alkyl, Aminoalkyl, -C(O)OCH₃, sowie x=1, 2, 3 oder 4,
wobei besonders bevorzugt R⁸= Alkenyl, insbesondere Vinyl, oder -(CH₂)ₙ-Z mit Z=NHR und n=1 oder 3, insbesondere n=1, und/oder x=3 und/oder R= -C(O)OCH₃.

Besonders bevorzugt ist das wenigstens eine funktionelle Alkoxysilan g) Vinyltrimethoxysilan, N-Trimethoxysilylmethyl-O-methylcarbamat und/oder eine Verbindung der folgenden Formel: 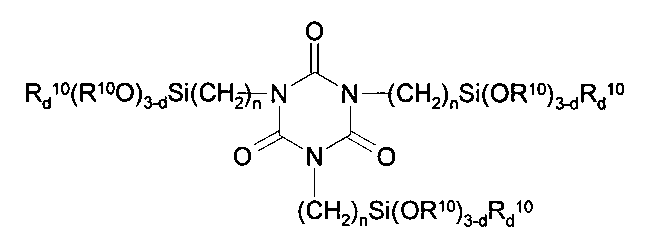 worin n = 1 bis 6, bevorzugt n = 1 oder 3, besonders bevorzugt n = 1, d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind.

Die zuvor genannten Verbindungen sind reaktive Silane, die als Wasserfänger zur Beseitigung von in der Komponente A der Dentalzusammensetzung noch vorhandenen Wasserspuren fungieren.

Des weiteren enthalten die erfindungsgemäßen Zweikomponenten-Dentalmaterialien vorzugsweise, und zwar wenn als Zweikomponentensystem formuliert besonders bevorzugt in der Katalysatorkomponente B, wenigstens einen Pastenbildner h), da dieser die Einstellung einer pastenartigen Konsistenz, bspw. dünn-, mittel- oder zähfließend, sowie eine homogene Vermischung des Salzes und der festen verstärkenden und nichtverstärkenden Füllstoffen ermöglicht. Vorzugsweise wird als wenigstens ein Pastenbildner h) eine Verbindung ausgewählt aus der Gruppe, welche aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden, Glycerin und Poly(meth)acrylsäuren besteht, eingesetzt. Selbstverständlich können die erfindungsgemäßen Dentalmaterialien auch eine beliebige Kombination zweier oder mehrerer der zuvor genannten Verbindungen enthalten.

Besonders bevorzugt sind hydrophile Pastenbildner, in denen sich das erfindungsgemäße Katalysatorsalz mit Wasser homogen mischen lässt. Die Mischbarkeit kann durch Zusatz von Tensiden noch verbessert werden. Besonders bevorzugte Vertreter sind Polyether, Polyurethane, Polyester, mehrwertige Alkohole, insbesondere Propylenglycole, Polypropylenglycole, Ethylenglycole, Polyethylenglycole, Butylenglycole, Polybutylenglycole und Glycerin, sowie Mischungen und Copolymere hiervon.

Bei den ggf. als Tensid, Emulgator und/oder Stabilisator eingesetzten Verbindungen i) handelt es sich vorzugsweise um anionische Tenside, besonders bevorzugt Alkylsulfate, Alkylbenzolsulfonate oder Alkylbenzolphosphate, kationische Tenside, besonders bevorzugt Tetraalkylammoniumhalogenide, nichtionische Tenside, besonders bevorzugt Alkyl- und Alkylphenyl-Polyalkylalkylenoxide, Fettsäurealkoxylate, Fettalkoholalkyloxylate sowie deren Alkylether und Alkylester, Fettsäurealkylolamide, Saccharosefettsäureester, Trialkylaminoxide, Silicontenside (z.B. Silwet L77, Tegopren 5878) oder Fluortenside, oder um amphotere Tenside, besonders bevorzugt sulfatierte oder oxyethylierte Kondensationsprodukte aus Alkenylphenolen und Formaldehyd, Ethylenoxid-propylenoxid-Blockpolymerisate oder modifizierte Polysiloxane. Mit Vorteil können auch in den alkoxysilylfunktionellen Polyether a) einpolymerisierbare Tenside, wie sie in der US 4,160,778, die hiermit als Referenz eingeführt und als Teil der Offenbarung gilt, offenbart sind, eingesetzt werden. Zusätzlich oder alternativ dazu können auch Derivate der zuvor genannten Tenside eingesetzt werden, bspw. solche, die über funktionelle Gruppen, wie -OH, -CH=CH₂, -OCO-(CH₃)C=CH₂ sowie Alkoxysilylgruppen, verfügen. Zudem können, wenn auch weniger bevorzugt, andere, dem Fachmann bekannte Tenside eingesetzt werden.

Bevorzugt wird als eingesetzten Verbindungen i) ein Silicontensid eingesetzt, da sich im Rahmen der vorliegenden Erfindung gezeigt hat, dass mit diesen Verbindungen überraschenderweise in der Polyethermatrix sehr niedrige, mit der Methode "liegender Tropfen" bestimmte, Kontaktwinkel erzielt werden können.

Diese Mischungen zeichnen sich durch eine ausgezeichnete Benetzbarkeit und ein hervorragendes Anfließverhalten an feuchte Zahn- und Gewebssubstanz aus. Trotz dieser guten hydrophilen Eigenschaften quillt das Material nicht bei Kontakt mit wässrigen Medien, wie Wasser, Speichel, Blut, Desinfektionsbad oder wässrigem Gipsbrei. Die gute initiale Benetzbarkeit der Mischungen ist wichtig für die detailgetreue Abformung des Abformmaterials in dem Patientenmund während der Verarbeitung und dem ersten Kontakt mit feuchter Mund-/Zahnsubstanz und drückt sich durch einen niedrigen Kontaktwinkel von kleiner 50°, insbesondere kleiner gleich 45°, gemessen mit einem Kontaktwinkelmessgerät der Firma Krüss bei 20 °C mit der Messmethode "liegender Tropfen", aus. Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 60°, insbesondere kleiner gleich 55°, aus.

Zudem können die erfindungsgemäßen Dentalmaterialien eine oder mehrere Wirkstoffe j) enthalten, welche in Abhängigkeit von deren chemischen Funktionalität bei Formulierung als Zweikomponentensystem in der Basiskomponente A oder der Katalysatorkomponente B enthalten sein können. Zu den erfindungsgemäß einzusetzenden Wirkstoffen zählen insbesondere Adstringentien, wie Epinephrine, antibakteriell und/oder antifugal wirkende Substanzen, wie Hexitidine (bspw. 5-Amino-1, 3-bis(2-ethylhexyl)-5-methylhexahydropyrimidin), Triclosane (bspw. 2,4,4'-Trichloro-2-hydroxydiphenylether) und Chlorhexidin: 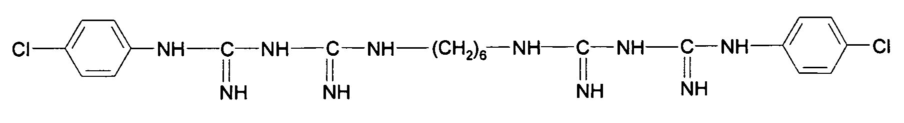 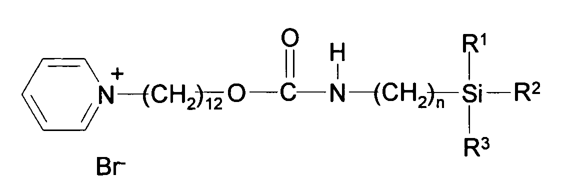 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

Als Weichmacher k) kommen insbesondere nicht reaktive Polyether, Polyester, Polyurethane, Phthalate, Mono-, Di-, Tri- oder höherwertige Ester, insbesondere Acetyltributylcitrat, Mesamoll® (Bayer) und Triglyceride in Betracht, welche bei Formulierung als Zweikomponentensystem je nach deren chemischen Natur der Komponente A und/oder der Komponente B zugesetzt werden.

Als die optische Lesbarkeit/Abtastung ermöglichenden Verbindungen I) können alle dem Fachmann zu diesem Zweck bekannte Substanzen, insbesondere Metallpulver, Metallpigmente, Metallicpigmente, Zinkoxid, Zirkonoxid und Titandioxid, eingesetzt werden, welche bei Formulierung als Zweikomponentensystem je nach deren chemischen Natur der Komponente A und/oder der Komponente B zugesetzt werden.

Zudem können die erfindungsgemäßen Dentalmaterialien in einer der beiden oder in beiden Komponenten übliche Geschmacks- und/oder Geruchsstoffe m) und/oder für die Diagnostik nützliche Zusätze n) enthalten, wie sie bspw. in der EP 1 339 373, PCT/EP00/05418 und DE 100 61 195 beschrieben sind.

Als Fluoridierungshilfsstoffe o) haben sich insbesondere Natriumfluorid, Kaliumfluorid, Ammoniumfluorid, Fluorphosphate und Aminfluoride, wie N'-Octadecylbimethylendiamin-N, N, N'-bis(2-ethanol)-dihydrofluorid (wie in ZM 93, Nummer 15, Seite 32 ff. beschrieben), als geeignet erwiesen, welche bei Formulierung als Zweikomponentensystem ebenfalls in Abhängigkeit von deren chemischen Natur der Komponente A und/oder der Komponente B zugesetzt sein können.

Zudem kann das erfindungsgemäße Dentalmaterial, bei Formulierung als Zweikomponentensystem in der Komponente A und/oder der Komponente B, als Bleichsubstanz p) eine oder mehrere verschiedene Peroxide enthalten, welche vorzugsweise aus der Gruppe, welche aus Alkalimetall- und Erdalkalimetallperoxiden, Wasserstoffperoxid sowie Carbamidperoxid besteht, ausgewählt sind.

Beispiele für geeignete Desensibilisierungsmittel q) sind Kaliumsalze, wie Kaliumnitrat, Nelkenöl und Eugenol.

Als Haftverbundvermittler r), bspw. zur Ausbildung eines Haftverbundes zwischen dem Abformmaterial und einem Abformlöffel aus Edelstahl und/oder Kunststoff, eignen sich insbesondere Alkoxysilane, Epoxysilane, Aminosilane und Methacrylatsilane.

Beispiele für geeignete Farbstoffe s) sind Farbstoffpigmente in Form von Al-, Ca-, Ba-Oxiden/verlackter Farbstoff, welche wie die zuvor beschriebenen Hilfsstoffe, sofern nicht anders angegeben, bei Formulierung als Zweikomponentensystem in Abhängigkeit von deren chemischen Natur der Komponente A und/oder der Komponente B zugesetzt sein können.

Des weiteren können den erfindungsgemäßen Dentalmaterial bei Formulierung als Zweikomponentensystem in der Komponente A und/oder der Komponente B Farbstoffindikatoren t) zugesetzt sein, welche ihre Farbe in Abhängigkeit von dem pH-Wert, bspw. aufgrund von pH-Wert-Änderungen beim Vermischen der Komponenten A und B, oder beim Kontakt mit Wasser ändern.

Als Stabilisatoren und/oder Antioxidantien u) können den erfindungsgemäßen Zweikomponenten-Dentalmaterialien insbesondere aus der aus polymerem Trimethyl-dihydrochinolin, Diphenylderivaten, Phenothiazin, Phenyl-α-naphthylamin, 4, 4'-Methylen-bis-2,6-di-tert.-butylphenol, Butylhydroxytoluol, Butylhydroxyanisol (BHA) und Methoxyphenol (Hydroxyanisol) bestehenden Gruppe ausgewählte Verbindungen eingesetzt werden. Beispiele für derartige Verbindungen sind die von der Firma Ciba-Geigy kommerziell erhältlichen Produkte Irganox 1010, 1076, 1035, MD 1024, Irgafos 168, 38, Irgacor 252 LD/252FC, 1405, 1930, 153, Tinuvin 328, P, 384, 900, 928, 327, 1130, 400, 292, 144, 123, 622 sowie Chimassorb 119.

Vorzugsweise wird das erfindungsgemäße Zweikomponenten-Dentalmaterial in geeigneten Primärverpackungen, wie Tuben, Dosen, und besonders bevorzugt in Kartuschen und Schlauchbeuteln, wie sie bspw. in der EP 0 723 807 A2, EP-A-0 541 972, PCT/EP/980193, EP-A-0 492 412, EP-A-0 492 413 und EP 0 956 908 A1, welche hiermit als Referenz eingeführt und somit als Teil der Offenbarung gelten, beschrieben sind, gelagert und auf die spätere Verwendung zugeschnitten proportioniert ist.

Ein weiterer Gegenstand der vorliegenden Erfindung sind Mischungen, welche durch Vermischen der Komponenten A und B des zuvor beschriebenen erfindungsgemäßen Zweikomponenten-Dentalmaterials erhältlich sind. Vorzugsweise wird die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1 oder 5:1, vermischt.

Im Folgenden wird die Erfindung anhand von den Erfindungsgedanken demonstrierenden, diesen aber nicht einschränkenden Beispielen erläutert.

### Beispiele 1 bis 6 (erfindungsgemäß)

### (Herstellung verschiedener Katalysatorkomponenten B mit unterschiedlichen erfindungsgemäß einzusetzenden Katalysatorsalzen aus starken Säuren und Pyridin)

### Herstellung verschiedener Katalysatorkomponenten B

Verschiedene Katalysatorsalze gebildet aus den in der Tabelle 1 angegebenen Mengen starker Säuren und Pyridin wurden in 2,9 Teilen entmineralisiertem Wasser (Ampuwa, pH 5,8) gelöst. Anschließend wurden die einzelnen Salzlösungen in einem Vakuummischbecher mit 36 Teilen Polypropylenglycol mit einer mittleren Molmasse von 4000 g/mol, 50 Teilen Quarzmehl mit einer mittleren Korngröße von 7 µm und 6 Teilen hochdisperser, hydrophobierter Kieselsäure mit einer BET-Oberfläche von 170 m²/g für 5 Minuten gemischt. Danach wurde weitere 30 Minuten unter Vakuum homogen gemischt.

Es wurden mittelfließende Materialien (ISO 4823) erhalten, die verschiedene Katalysatorkomponenten B des erfindungsgemäßen Abformmaterials auf Basis von Alkoxysilylpolyethern darstellen. Die Materialien wurden in Schlauchbeutel (Verbundfolie PE/AI/PE) abgefüllt und aufbewahrt. Diese wiesen pH-Werte im Bereich von pH 1 bis pH 3 auf.

### Herstellung einer Basiskomponente A

In einem Vakuummischer wurden unter trockener Argon-Schutzgasatmosphäre 40 Teile eines Polypropylenglycols, das endständig über Urethangruppen und Methylenspacer mit Dimethoxymethylsilylgruppen funktionalisiert war, wobei das funktionalisierte Polypropylenglycol eine Viskosität bei 20°C von 10.000 mPa·s aufwies, mit 50 Teilen eines getrockneten mit Trimethylsilylgruppen oberflächenmodifizierten Cristobalitfüllstoffs mit einer mittleren Korngröße von 7 µm, 7 Teilen einer getrockneten, hochdispersen, pyrogen hergestellten, hydrophobierten Kieselsäure mit einer BET-Oberfläche von 170 m²/g, 0,8 Teilen Vinyltrimethoxysilan und 2 Teilen Silicontensid für 5 Minuten gemischt. Danach wurde weitere 30 Minuten unter Vakuum homogen gemischt.

Es wurde ein mittelfließendes Material (ISO 4823) erhalten, das die Basiskomponente A des erfindungsgemäßen Abformmaterials auf Basis von Alkoxysilylpolyethern darstellt. Das Material wurde in Schlauchbeutel (Verbundfolie PE/AI/PE) abgefüllt und aufbewahrt.

### Mischung der Katalysatorkomponenten B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts (Plug & Press System, Kettenbach GmbH & Co. KG) aus Schlauchbeuteln über einen dynamishen Mischer homogen gemischt.

Die Abbindezeiten der so hergestellten Dentalmaterialien auf Basis von Alkoxysilylpolyethern sind in der Tabelle 1 sowie weitere anwendungstechnische Eigenschaften für das in Beispiel 2 erhaltene Dentalmaterial sind in den Tabellen 3 und 4 zusammengefasst.

Die Beispiele 1 bis 6 zeigen, dass die erfindungsgemäße Zusammensetzungen überraschenderweise eine für kondensationsvernetzende Systeme hervorragende Aushärtekinetik aufweisen. Es lassen sich schnellabbindende Abformmaterialien mit praxisgerechten Verarbeitungszeiten herstellen. Der Fachmann erkennt, dass die Abbindezeit der einzelnen Beispiele durch Erhöhung bzw. Reduzierung der Menge an eingesetzten Katalysatorsalz einfach auf einen gewünschten Wert eingestellt werden kann.

Wie insbesondere aus der Tabelle 4 für die in Beispiel 2 erhaltene Masse hervorgeht erfüllen die erfindungsgemäßen Dentalmaterialien alle Anforderungen für ein funktionsfähiges dentales Abformmaterial, vor allem im Hinblick auf Shore A-Härten, Rückstellung nach der Verformung, Konsistenzen der Einzelkomponenten und der Mischung, lineare Maßänderungen und Kontaktwinkel. Insbesondere werden für die durch Kontaktwinkelmessung bestimmte Hydrophilie hervorragende Werte erhalten.

### Vergleichsbeispiel 1 (nicht erfindungsgemäß)

Ein Salz gebildet aus den in der Tabelle 1 angegebenen Mengen Pyridin und Essigsäure wurde in 5,0 Teilen entmineralisiertem Wasser (Ampuwa, pH 5,8) gelöst und gleichermaßen wie in den Beispielen 1 bis 6 zu einer Katalysatorkomponente B verarbeitet.

Ein Teil der zuvor beschriebenen Katalysatorkomponente B und 5 Teile der Basiskomponente A gemäß Beispiel 1 wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeit des so hergestellten Materials auf Basis von Alkoxysilylpolyether ist in der Tabelle 1 zusammengefasst.

Wie aus der Tabelle 1 ersichtlich weist das in Vergleichsbeispiel 1 eingesetzte Salz gebildet aus Pyridin und einer schwachen Säure mit einem pKs-Wert von 4,76 (Essigsäure) im Unterschied zu den erfindungsgemäßen Formulierungen eine inakzeptable Abbindezeit von mehr als 60 Minuten auf.

### Beispiele 7 bis 13 (erfindungsgemäß)

### (Herstellung verschiedener Katalysatorkomponenten B mit unterschiedlichen erfindungsgemäß einzusetzenden Katalysatorsalzen aus starken Säuren und schwachen Basen)

### Herstellung verschiedener Katalysatorkomponenten B

Verschiedene Katalysatorsalze gebildet aus den in der Tabelle 2 angegebenen Mengen starker Säuren und schwacher Basen wurden in 2,9 Teilen entmineralisiertem Wasser (Ampuwa, pH 5,8) gelöst. Anschließend wurden die einzelnen Salzlösungen in einem Vakuummischbecher mit 36 Teilen Polypropylenglycol mit einer mittleren Molmasse von 4000 g/mol, 50 Teilen Quarzmehl mit einer mittleren Korngröße von 7 µm und 6 Teilen hochdisperser, hydrophobierter Kieselsäure mit einer BET-Oberfläche von 170 m²/g für 5 Minuten gemischt. Danach wurde weitere 30 Minuten unter Vakuum homogen gemischt.

Es wurden mittelfließende Materialien (ISO 4823) erhalten, die verschiedene Katalysatorkomponenten B des erfindungsgemäßen Abformmaterials auf Basis von Alkoxysilylpolyethern darstellen. Die Materialien wurden in Schlauchbeutel (Verbundfolie PE/AI/PE) abgefüllt und aufbewahrt.

### Mischung der Katalysatorkomponenten B und der Basiskomponente A aus Beispiel 1

Jeweils 1 Teil der zuvor beschriebenen Katalysatorkomponente B und 5 Teile der gemäß Beispiel 1 hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Dentalmaterialien auf Basis von Alkoxysilylpolyethern sind in der Tabelle 2 zusammengefasst.

Die Beispiele 7 bis 13 zeigen, dass die erfindungsgemäße Zusammensetzungen überraschenderweise eine für kondensationsvernetzende Systeme hervorragende Aushärtekinetik ermöglicht. Es lassen sich schnellabbindende Abformmaterialien mit praxisgerechten Verarbeitungszeiten herstellen. Der Fachmann erkennt, dass die Abbindezeit der einzelnen Beispiele durch Erhöhung bzw. Reduzierung der Menge an eingesetzten Katalysatorsalz einfach auf einen gewünschten Wert eingestellt werden kann.

### Vergleichsbeispiele 2 und 3 (nicht erfindungsgemäß)

Zwei verschiedene Salze gebildet aus den in der Tabelle 2 angegebenen Mengen p-Toluolsulfonsäure und verschiedenen Basen wurden in 5,0 Teilen entmineralisiertem Wasser (Ampuwa, pH 5,8) gelöst und gleichermaßen wie in den Beispielen 1 bis 6 zu einer Katalysatorkomponente B verarbeitet.

Jeweils 1 Teil der zuvor beschriebenen Katalysatorkomponenten B und 5 Teile der Basiskomponente A gemäß Beispiel 1 wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt.

Die Abbindezeiten der so hergestellten Materialien auf Basis von Alkoxysilylpolyethern sind in der Tabelle 2 zusammengefasst.

Wie aus der Tabelle 2 ersichtlich härten die in den Vergleichsbeispielen eingesetzten Materialien enthaltend Salze, deren Basenkomponente einen außerhalb des erfindungsgemäß einzuhaltenden pK_{BH+}-Bereich von -1 bis 7 liegenden Wert aufweisen, nicht aus.

### Vergleichsbeispiel 4 (nicht erfindungsgemäß)

### (Säurekatalysiertes kondensationsvernetzendes Dentalmaterial auf Basis von Alkoxysilylpolyethern gemäß Beispielen 3 und 5 der EP 1 226 808 A2)

Ein säurekatalysiertes Dentalmaterial nach dem Stand der Technik auf Basis von Alkoxysilypolyethern bestehend aus einer sauren Katalysatorkomponente B und einer Basiskomponente A wird gemäß Beispielen 3 und 5 der EP 1 226 808 A2 hergestellt und gemischt.

Die Verarbeitungszeit, Abbindezeit und die Abbindezeit nach einem Thermostresstest von einer Woche bei 60°C der gemäß Vergleichsbeispiel 4 hergestellten Zusammensetzung im Vergleich zu dem in Beispiel 2 erhaltenen erfindungsgemäßen Dentalmaterial auf Basis von Alkoxysilylpolyethern sind in der Tabelle 3 zusammengefasst.

Im Vergleich zu dem in Beispiel 2 erhaltenen, erfindungsgemäßen Dentalmaterial auf Basis von Alkoxysilylpolyethern zeigt das säurekatalysierte System aus EP 1 226 808 A2 (Beispiele 3 und 5) nach Lagerung im Thermostresstest (eine Woche 60 °C) keine Aushärtung mehr. Dies zeigt, dass die Katalysatorkomponente aus Beispiel 3 der EP 122 6088 A2 instabil ist. Dies führt zu einer Verlängerung der Abbindezeit. Eine unabhängig von der Lagerzeit gleich bleibende Abbindezeit ist allerdings eine der wichtigsten Anforderungen an ein dentales Abformmaterial.

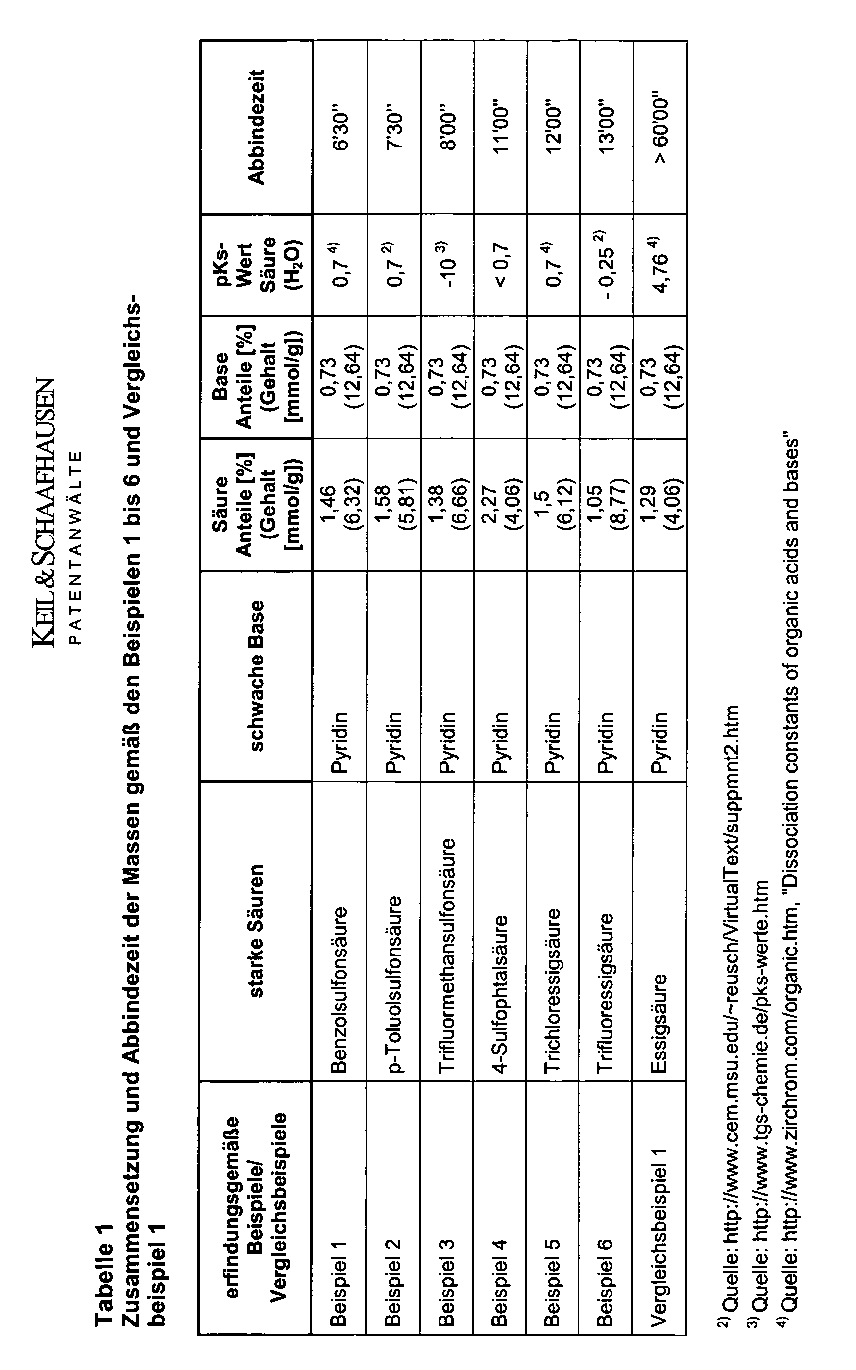

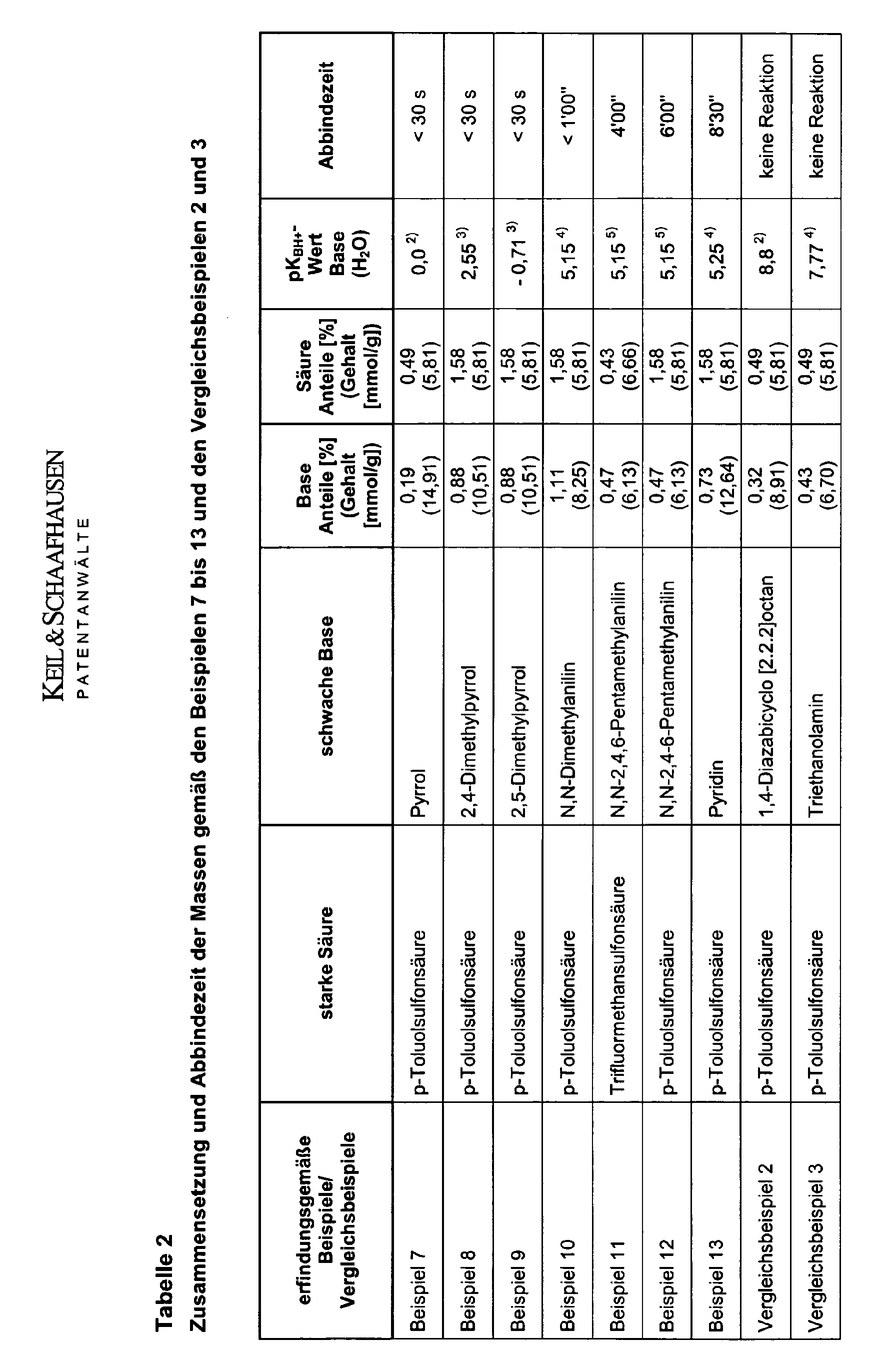

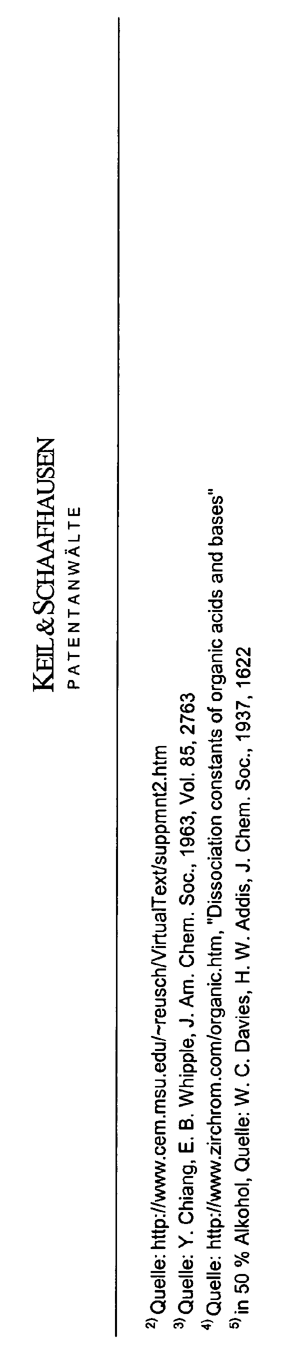

**Tabelle 3**

| **Verarbeitungs-, Abbindezeiten und Stabilitäten von Abformmaterialien auf Basis Alkoxysilylpolyether im Vergleich zu den Beispielen 3 und 5 der EP 1 226 808 A2** | | | |
|---|---|---|---|
| **Abformmaterial:** | **Verarbeitungszeit** ^{**1)**} | **Abbindezeit** ^{**2)**} | **Stabilität: Abbindezeit nach einer Woche 60°C** ^{**3)**} |
| Beispiel 2 | 3,00 min | 7,50 min | 7,75 min |
| Vergleichsbeispiel 4 (Beispiel 3 und 5 EP 122 6808 A2) | 0,50 min | 5,00 min | > 15,00 min |

| | | | |
|---|---|---|---|
| ¹⁾ gemäß ISO 4823 | | | |
| ²⁾ Die Abbindezeit wurde bestimmt über Rückstellung nach der Verformung nach ISO 4823 (Ausgabe 1992) | | | |
| ³⁾ Einlagerung in verschlossenen Schlauchbeuteln aus PE/AI/PE-Verbundfolie, Messung siehe unter ²⁾ | | | |

**Tabelle 4**

| **Anwendungstechnische Eigenschaften der Zusammensetzungen gemäß Beispiel 2** | |
|---|---|
| | **Beispiel 2 Katalysatorsalz aus p-Toluolsulfonsäure mit Pyridin** |
| **Konsistenz** ^{**1)**} **Katalysatorkomponente B** | 30 |
| **Konsistenz** ^{**1)**} **Basiskomponente A** | 33 |
| **Konsistenz der Mischung**^{**2)**} | **36** |
| **Lineare Maßänderung** ^{**3)**} | - 0,60 % |
| **Shore A-Härte sofort nach Abbindeende** ^{**4)**} | 30 |
| **Shore A-Härte sofort nach 15 Stunden Lagerung** ^{**4)**} | 60 |
| **Kontaktwinkel** ^{**5)**} | 30°/40° |
| **Rückstellung nach der Verformung** ^{**6)**} | 98,05 % |

| | |
|---|---|
| ¹⁾ in Anlehnung an ISO 4823, Konsistenz der Mischung, Belastungsgewicht 500 g, Belastungsdauer 15 s | |
| ²⁾ gemäß ISO 4823, Konsistenz der Mischung, Belastungsgewicht 1500 g, Belastungsdauer 5 s | |
| ³⁾ gemäß ISO 4823 | |
| ⁴⁾ gemäß DIN 53505 mit digitalem Shore-Härteprüfgerät Fa. Zwick | |
| ⁵⁾ gemessen nach der Methode des liegenden Tropfens, Kontaktwinkelmeßsystem Fa. Krüss G40, initialer Kontaktwinkel (Prüfkörperalter: Aufbringen des Tropfens 45 Sek. nach Mischbeginn), Messzeitpunkt: 30 Sek. nach Aufbringen des Tropfens, Verwendung von entmineralisiertem Wasser. | |
| ⁶⁾ gemäß ISO 4823 | |

## Patentansprüche

1. Kondensationsvernetzendes Dentalmaterial, insbesondere Dentalabformmaterial, enthaltend:
a) wenigstens einen alkoxysilylfunktionellen Polyether und
b) wenigstens einen Katalysator
**dadurch gekennzeichnet, dass** der wenigstens eine Katalysator b) ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2.

2. Kondensationsvernetzendes Zweikomponenten-Dentalmaterial, insbesondere Dentalabformmaterial, mit einer Komponente A enthaltend
a) wenigstens einen alkoxysilylfunktionellen Polyether
und einer Komponente B enthaltend
b) wenigstens einen Katalysator und
c) Wasser,
wobei der wenigstens eine Katalysator b) ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2.

3. Kondensationsvernetzendes Dentalmaterial nach Anspruch 1 oder, **dadurch gekennzeichnet, dass** das der wenigstens eine Katalysator b) ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2, wobei die Säure eine Struktur aufweist, welche nach Deprotonierung der Säure eine Mesomeriestabilisierung der negativen Ladung ermöglicht.

4. Kondensationsvernetzendes Dentalmaterial nach einem der vorhergehenden Ansprüche, **dadurch gekennzeichnet, dass** dieses wenigstens einen verstärkenden Füllstoff d₁) mit einer BET-Oberfläche von mindestens 50 m²/g und/oder wenigstens einen nicht verstärkenden Füllstoff d₂) mit einer BET-Oberfläche von weniger als 50 m²/g enthält.

5. Dentalmaterial nach einem der vorhergehenden Ansprüche, **dadurch gekennzeichnet, dass** das wenigstens eine eingesetzte Katalysatorsalz gebildet ist aus wenigstens einer Base mit einem in Wasser gemessenen pK_{BH+}-Wert zwischen 1 und 7, besonders bevorzugt zwischen 2 und 6 und ganz besonders bevorzugt zwischen 3 und 6 und/oder wenigstens einer Säure mit einem in Wasser gemessenen pKs-Wert von kleiner 1 und besonders bevorzugt von kleiner gleich 0,7.

6. Dentalmaterial nach einem der vorhergehenden Ansprüche, **dadurch gekennzeichnet, dass** der wenigstens eine Katalysator ein Salz ist gebildet aus einer aus der aus p-Toluolsulfonsäure, Fluorsulfonsäure, Trifluormethansulfonsäure, Fluoroschwefelsäure, 4-Sulfophthalsäure, Trichloressigsäure, Trifluoressigsäure, Benzolsulfonsäure und Kombinationen hiervon bestehenden Gruppe ausgewählten Säure und/oder einer aus der aus Pyrrolderivaten, Dimethylanilin, Pyridin, 2,4,6-N,N-Pentamethylanilin, N,N-Dimethylanilin, Phenetedin, Acridin, Phenanthridin, Chinolin, Isochinolin, 2-Amino-4,6-dimethylpyrazin, 4,6-Dimethylpyridinamin, 3-Methylpyridin(3-picolin), 4-Phenyl-pyridin, 4-Vinylpyridin, Pyridazin, 2-Ethylpyridin, 2-Butylpyridin, 1,7-Phenanthrolin, 2-Aminopyrimidin, 2-Isopropylpyridin, 2-Vinylpyridin, 2-N,N-Dimethyl-aminopyridin, Chinazolin, 4-Chloropyridin, Phenazin, 4-Acetylpyridin, Methylnicotinat, 3-Benzoylpyridin, 2,2'-Bipyridin, 2-Phenylpyridin, 2-tert-Butylpyridin, Pyrimidin, 3-lodopyridin, 3-Fluoropyridin, 3-Chloropyridin, 3-Bromopyridin, Pyrazin, 7,8-Benzochinolin, 2-Chloropyridin, 4-Cyanopyridin und Kombinationen hiervon bestehenden Gruppe ausgewählten Base.

7. Dentalmaterial nach einem der vorhergehenden Ansprüche, **dadurch gekennzeichnet, dass** dieses, bezogen auf die Gesamtmischung, 0,0005 bis 0,5 mmol/g, besonders bevorzugt 0,0005 bis 0,25 mmol/g und ganz besonders bevorzugt 0,0005 bis 0,05 mmol/g Katalysatorsalz enthält.

8. Dentalmaterial nach einem der vorhergehenden Ansprüche, **dadurch gekennzeichnet, dass** das wenigstens eine Katalysatorsalz in der Polyethermatrix, vorzugsweise eine Polyethermatrix umfassend als Struktureinheit Polytetrahydrofuran, Polyethylenglycol und besonders bevorzugt Polypropylenglycol und Mischungen und Copolymerisate hiervon, eine hinreichend große Löslichkeit aufweist, um in einer auf die Gesamtmischung bezogenen Menge von 0,0005 bis 0,5 mmol/g eingesetzt eine Aushärtung des Dentalmaterials bestimmt durch Rückstellung nach Verformung gemäß ISO 4823 (Ausgabe 1992) in weniger gleich 30 Minuten, bevorzugt weniger gleich 15 Minuten für eine Dubliermasse und in weniger gleich 15 Minuten, besonders bevorzugt weniger gleich 10 min, ganz besonders bevorzugt weniger gleich 7 min und höchst bevorzugt weniger gleich 6 min für eine dentale Abformmasse zu erzielen.

9. Dentalmaterial nach einem der vorhergehenden Ansprüche, **dadurch gekennzeichnet, dass** dieses neben einem oder mehreren Salzen gemäß einem der Ansprüche 1 bis 8 keinen weiteren Katalysator, insbesondere keine metallorganischen Verbindungen, keine Schwermetallcarboxylatsalze, tertiären Amine und freien Säuren, enthält.

10. Dentalmaterial nach einem der vorhergehenden Ansprüche, **dadurch gekennzeichnet, dass** der wenigstens eine alkoxysilylfunktionelle Polyether a) als dritte Struktureinheit jeweils an den terminalen Alkoxysilylgruppen angeordnete Alkylenspacer, welche besonders bevorzugt C₁-C₆-Alkylgruppen, ganz besonders bevorzugt C₁-C₃-Alkylgruppen und höchst besonders bevorzugt Ethylengruppen (C₂) und/oder Methylengruppen (C₁) sind, und als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweist, wobei solche Alkoxysilylpolyether höchst besonders bevorzugt sind, die innerhalb der Polymerkette keine Urethan- und/oder Harnstoffgruppen enthalten und die an jedem Kettenende höchstens eine bzw. nicht mehr als eine Urethan und/oder Harnstoffgruppe und höchstens eine bzw. nicht mehr als eine Methylenspacergruppe tragen, wobei die einzelnen Struktureinheiten des wenigstens einen Polyethers a) vorzugsweise gemäß 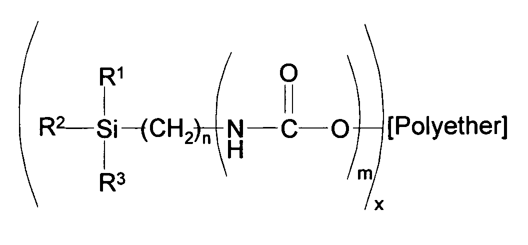 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist/sind
und/oder 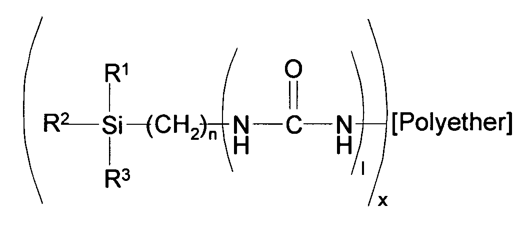 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind, sowie
x=1 bis 6, bevorzugt x=2 bis 4 und ganz besonders bevorzugt x=2, n=1 bis 6, bevorzugt n=1 bis 3 und ganz besonders bevorzugt n=1
sowie I=0 oder 1, besonders bevorzugt I=1 ist/sind,
angeordnet sind.

11. Dentalmaterial nach Anspruch 10, **dadurch gekennzeichnet, dass** n gleich 1 ist.

12. Dentalmaterial nach einem der vorhergehenden Ansprüche, **dadurch gekennzeichnet, dass** der wenigstens eine Polyether a) eine aus der aus Polytetrahydrofuran, Polyethylenglycol, Polypropylenglycol, Copolymerisaten hiervon und Mischungen hiervon bestehenden Gruppe ausgewählte Struktureinheit enthält.

13. Dentalmaterial nach einem der vorhergehenden Ansprüche, **dadurch gekennzeichnet, dass** dieses des weiteren wenigstens einen Pastenbildner h) enthält, welcher vorzugsweise aus der aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden und Poly(meth)acrylsäuren bestehenden Gruppe ausgewählt ist.

14. Dentalmaterial nach einem der vorhergehenden Ansprüche, **dadurch gekennzeichnet, dass** dieses des weiteren wenigstens einen Wasserfänger d) enthält, welcher vorzugsweise Vinyltrimethoxysilan, N-Trimethoxysilylmethyl-O-methylcarbamat und/oder eine Verbindung der folgenden Formel: 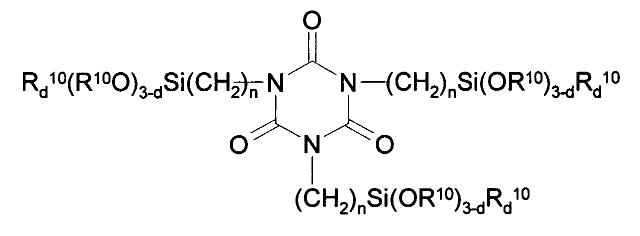 worin n = 1 bis 6, bevorzugt n = 1 oder 3, besonders bevorzugt n = 1, d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind,
ist.

15. Mischung erhältlich durch Vermischen der Komponenten A und B des Zweikomponenten-Dentalmaterials nach einem der Ansprüche 2 bis 14, **dadurch gekennzeichnet, dass** die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1 oder 5:1 vermischt wird.

16. Verwendung eines Dentalmaterials nach einem der Ansprüche 1 bis 15 in der Dentalmedizin und/oder Dentaltechnik.
